# FASCICULE DE BREVET EUROPEEN

(11) **EP 0 522 959 B1**
(45) Date de publication et mention de la délivrance du brevet: **26.03.1997**
(21) Numéro de dépôt: 92401964.9
(22) Date de dépôt: 08.07.1992
(51) Int. Cl.: G01N 1/10, G21F 7/005, G21F 7/06

(54) **Procédé et installation pour transférer en dehors d'une enceinte étanche un fluide contenu dans un récipient fermé**
Verfahren und Vorrichtung zum Transfer eines in einem geschlossener Behälter enthaltenen Fluidums aus einer dichten Umgebung
Method and apparatus for transferring fluid from a closed container out of a sealed enclosure

(30) Priorité: 10.07.1991 FR 9108686
(43) Date de publication de la demande: 13.01.1993
(73) Titulaire: COGEMA COMPAGNIE GENERALE DES MATIERES NUCLEAIRES, F-78141 Velizy-Villacoublay (FR)
(72) Inventeur: Ringot, Gilbert, F-50460 Querqueville (FR); Ferron, Denis, F-50120 Equeurdreville (FR)
(74) Mandataire: Poulin, Gérard

(56) Documents cités:
- EP-A- 0 078 212
- DE-A- 3 044 424
- FR-A- 2 041 587
- FR-A- 2 058 751
- US-A- 4 662 231

## Description

L'invention concerne un procédé pour transférer en dehors d'une enceinte étanche un fluide contenu dans un récipient fermé tel qu'un cruchon obturé par un opercule, sans sortir ce récipient de l'enceinte et sans rompre l'étanchéité de cette dernière. L'invention concerne également une installation mettant en oeuvre ce procédé.

Dans les usines de traitement de combustibles nucléaires irradiés, il est d'usage d'effectuer en différents points du procédé des prélèvements d'échantillons de solutions actives, en vue de réaliser des analyses et des mesures sur ces échantillons.

Comme cela est décrit dans le document FR-A-2 515 350, les prélèvements peuvent notamment être effectués dans des cruchons en matière plastique, qui sont placés dans des récipients appelés curseurs dont la forme particulière favorise le transfert des cruchons dans des tubes, sous l'action de moyens de transfert pneumatiques. Chaque cruchon est obturé par un opercule perforable.

Le document US-A-4 662 231 décrit un dispositif de prélèvement d'échantillons, dans lequel un cruchon est introduit pneumatiquement dans un logement prévu dans un barillet, amené à un poste de remplissage par une rotation du barillet, pour y recevoir un échantillon liquide en provenance d'un circuit extérieur, puis évacué pneumatiquement du logement après une nouvelle rotation du barillet. Le remplissage du cruchon est assuré lorsqu'un ensemble d'aiguilles relié au circuit extérieur a perforé un opercule du cruchon.

Lorsque la chaîne d'analyse appartient à la chaîne blindée à l'intérieur de laquelle s'effectue le prélèvement des échantillons, les cruchons contenant ces derniers peuvent être transférés directement dans cette chaîne d'analyse par les moyens de transfert pneumatiques précités, sans précaution particulière.

Au contraire, lorsque certaines mesures ou analyses sont effectuées à l'extérieur de la chaîne blindée dans laquelle sont reçus les échantillons, comme cela peut notamment être le cas pour certaines opérations de radiométrie ou certaines mesures physiques concernant des solutions faiblement actives, le transfert des échantillons hors de la chaîne blindée présente certaines difficultés, notamment lorsque les mesures à affectuer nécessitent que ce transfert s'effectue sans aucune dilution de l'échantillon prélevé.

En particulier, il est essentiel que le transfert des échantillons hors de la chaîne blindée s'effectue dans un conditionnement propre, ce qui n'est pas le cas des cruchons contenant les échantillons reçus à l'intérieur de la chaîne blindée, compte tenu de la pollution régnant dans cette dernière. Par ailleurs, le transfert des échantillons à l'extérieur de la chaîne blindée doit pouvoir s'effectuer sans polluer ces échantillons et sans que le nouveau conditionnement propre qui les reçoit ne soit contaminé lors du transfert. Il doit aussi être possible de vérifier que les échantillons dans leur nouveau conditionnement propre ne sont pas trop irradiants, si l'on désire que les analyses soient effectuées dans des boîtes à gants.

Par ailleurs, on connaît du document FR-A-2 041 587 un dispositif conçu pour introduire dans des flacons destinés à une application médicale un générateur de radioisotope en solution. Pour cela, une solution est transférée depuis un réservoir jusqu'à un flacon à remplir, en passant par une colonne de verre qui contient le générateur de radioisotope. Le transfert s'effectue par deux tubes souples terminés par des aiguilles qui traversent des opercules fermant la colonne et le flacon. Il est provoqué par une dépression initiale dans le flacon, dès que l'opercule de ce dernier est transpercé suite à la manoeuvre d'une poignée prévue à cet effet.

L'invention a précisément pour objet un procédé et une installation permettant de transférer en dehors d'une enceinte étanche, dans un nouveau conditionnement propre, des échantillons fluides initialement contenus dans des récipients pollués, sans sortir ces récipients de l'enceinte et sans rompre l'étanchéité de cette dernière.

Conformément à l'invention, ce résultat est obtenu au moyen d'un procédé pour transférer en dehors d'une enceinte étanche un fluide contenu dans un premier récipient, sans sortir ce récipient de l'enceinte et sans rompre l'étanchéité de cette dernière, consistant à :
- introduire un deuxième récipient sous vide dans un réceptacle mobile placé à l'intérieur de l'enceinte, par un tube traversant de façon étanche une paroi de l'enceinte et débouchant dans le réceptacle lorsque ce dernier occupe une première position ;
- placer le réceptacle mobile dans une deuxième position, dans laquelle une zone perforable du deuxième récipient est exposée à l'intérieur de l'enceinte, et perforer ladite zone, ainsi qu'une zone perforable du premier récipient au moyen des deux extrémités d'une même aiguille, de façon à réaliser un transfert automatique du fluide dans le deuxième récipient sous vide ; puis
- ramener le réceptacle mobile dans sa première position et évacuer le deuxième récipient hors de l'enceinte par ledit tube.

Avantageusement, on introduit le deuxième récipient dans le réceptacle mobile et on évacue le deuxième récipient hors de l'enceinte par des moyens de transfert pneumatiques.

L'invention a aussi pour objet une installation pour transférer en dehors d'une enceinte étanche un fluide contenu dans un premier récipient, sans sortir ce récipient de l'enceinte et sans rompre l'étanchéité de cette dernière, comprenant :
- une réceptacle mobile à l'intérieur d'un corps placé dans l'enceinte, entre une première position et une deuxième position ;
- un tube traversant de façon étanche une paroi de l'enceinte et raccordé sur le corps, de façon à déboucher dans le réceptacle lorsque ce dernier occupe sa première position, pour y introduire un deuxième récipient sous vide ;
- un trou formé dans ledit corps en un emplacement situé en face du réceptacle, lorsque ce dernier occupe sa deuxième position ;
- un porte aiguille portant une aiguille dont une première extrémité est apte à être plantée dans une zone perforable du premier récipient et dont une deuxième extrémité est apte à être plantée dans une zone perforable du deuxième récipient, au travers dudit trou, lorsque le réceptacle occupe sa deuxième position.

De préférence, le corps dans lequel est logé le réceptacle mobile supporte, en face du trou permettant le passage de l'aiguille, un organe de guidage sur lequel un coulisseau, apte à recevoir le porte aiguille, peut se déplacer selon une direction confondue avec l'axe du trou.

Afin de permettre l'évacuation du deuxième récipient, lorsque le fluide contenu dans le premier récipient y a été transféré, l'installation comporte avantageusement une tuyauterie, qui traverse de façon étanche ladite paroi de l'enceinte et comporte une première extrémité raccordée sur le corps, de façon à déboucher dans le réceptacle à l'opposé du tube et une deuxième extrémité raccordée sur des moyens d'introduction d'air, à l'extérieur de l'enceinte.

Le corps dans lequel est logé le réceptacle peut également comporter un orifice d'évacuation exceptionnelle du deuxième récipient, normalement obturé par un bouchon, en face duquel peut être amené le réceptacle, dans une troisième position de ce dernier. Un organe de blocage monté dans le corps empêche alors normalement le passage du réceptacle dans la troisième position, tant que cet organe de blocage n'est pas actionné.

Selon un mode de réalisation préféré de l'invention, le réceptacle mobile est monté dans un boisseau rotatif, d'axe perpendiculaire à un axe dudit réceptacle.

On décrira maintenant, à titre d'exemple non limitatif, un mode de réalisation préféré de l'invention, en se référant aux dessins annexés, dans lesquels :
- la figure 1 est une vue de côté, en coupe partielle, illustrant schématiquement une installation de transfert conforme à l'invention ;
- la figure 2 est une vue de côté, en coupe verticale, illustrant à plus grande échelle la partie de l'installation située à l'intérieur de l'enceinte étanche ;
- la figure 3 est une vue en coupe à plus grande échelle selon la ligne III-III de la figure 2 ;
- la figure 4 est une vue en coupe verticale comparable à la figure 2, illustrant encore à plus grande échelle l'étape de transfert du fluide entre les deux récipients.

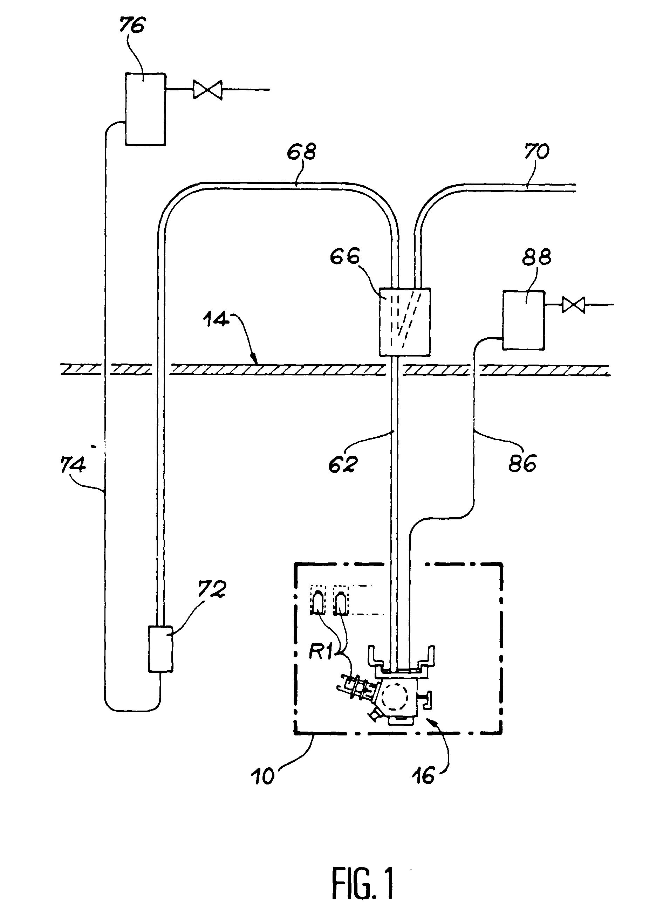

Sur la figure 1, la référence 10 désigne une enceinte étanche appartenant par exemple à une chaîne blindée dans laquelle sont effectués des analyses sur des solutions de combustibles nucléaires irradiés. A l'intérieur de cette enceinte se trouve placé un certain nombre de récipients R1 tels que des cruchons en matière plastique, dans lesquels ont été préalablement introduits des échantillons de solutions faiblement actives prélevés en différents points de l'usine. A titre d'illustration, ces prélèvements peuvent notamment avoir été effectués à l'aide d'unités de prélèvement telles que les unités décrites dans le document FR-A-2 515 350. Chacun des cruchons constituant les récipients R1 est obturé à l'une de ses extrémités par un opercule perforable, et placé dans un curseur cylindrique permettant son tranfert pneumatique vers la chaîne blindée.

L'enceinte étanche 10 est elle même placée dans une cellule de confinement 14 dont seule la paroi supérieure est illustrée sur la figure 1.

Conformément à l'invention, une installation est prévue afin de transférer en dehors de l'enceinte étanche 10 et de la cellule 14 qui la contient, chacun des échantillons fluides contenus dans les récipients pollués R1, sans sortir ces récipients de l'enceinte 10 et sans rompre l'étanchéité de cette dernière. Ce transfert peut notamment avoir pour objectif d'amener les échantillons fluides contenus dans chacun des récipients R1 dans des boîtes à gants (non représentées) situées à l'extérieur de la cellule 14 et permettant notamment d'effectuer des analyses de radiométrie ou des mesures physiques.

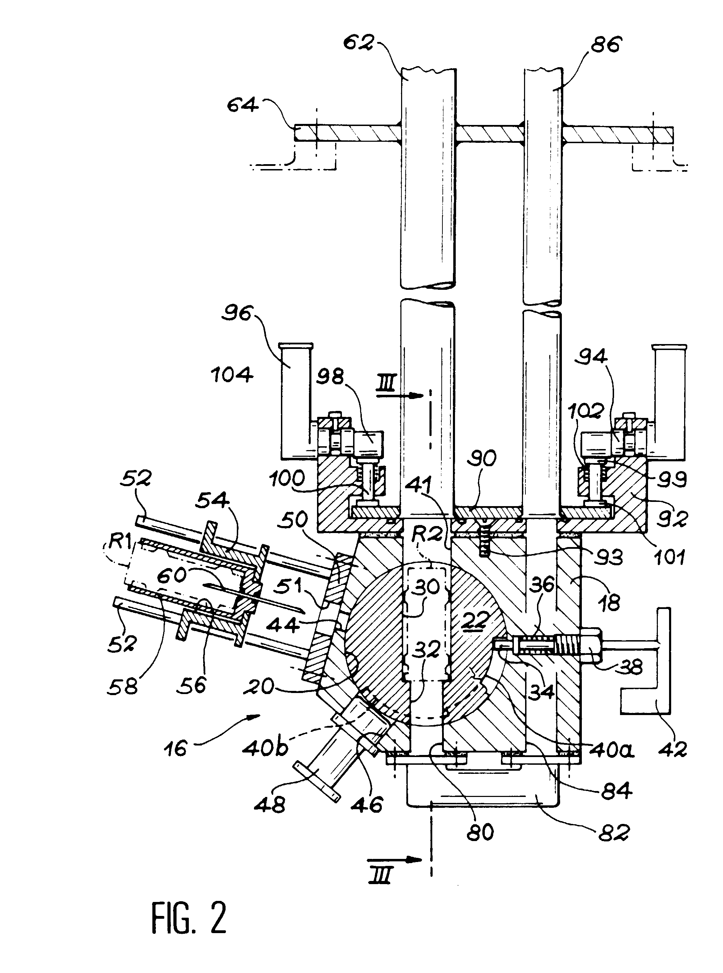

Cette installation est conçue pour réaliser successivement le transfert de chacun des échantillons fluides contenus dans les récipients pollués R1 dans des récipients neufs R2, non pollués (figure 2), qui peuvent être identiques aux récipients pollués, à l'intérieur même de l'enceinte étanche 10, puis le transfert de ces récipients neufs non pollués hors de l'enceinte étanche 10 puis de la cellule 14.

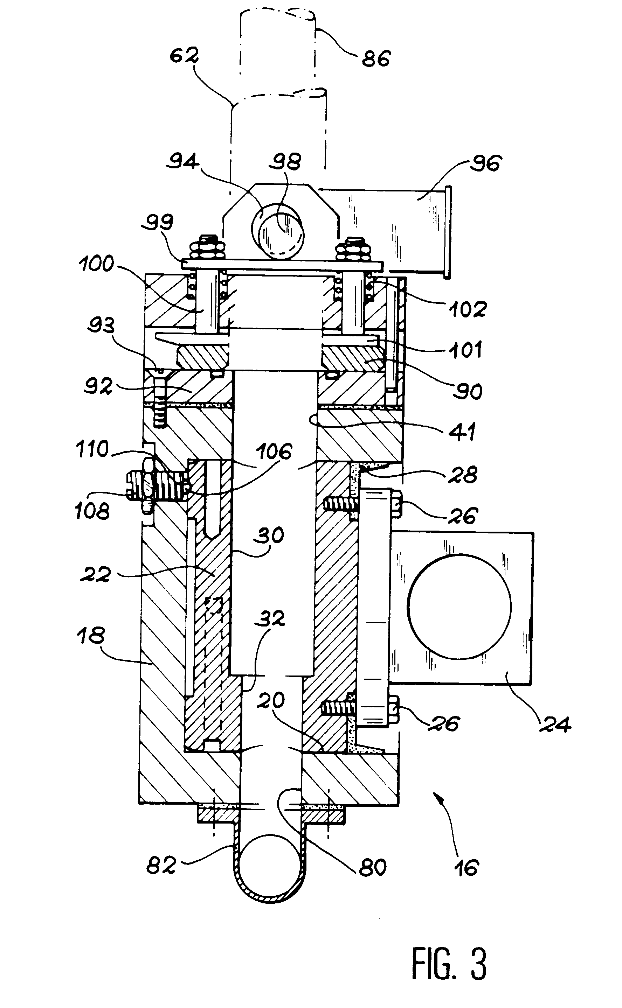

Pour réaliser le transfert entre les récipients R1 et R2, l'installation comprend principalement un appareil de transfert entre récipients placé à l'intérieur de l'enceinte étanche 10 et désigné de façon générale par la référence 16 sur la figure 1. Cet appareil 16 de transfert entre récipients va à présent être décrit en détail en se référant aux figures 2 et 3.

L'appareil 16 de transfert entre récipients, qui est placé à l'intérieur de l'enceinte étanche 10, comprend un corps 18, réalisé par exemple en matière plastique rigide. Ce corps 18 présente un évidement 20, de section verticale circulaire, qui débouche latéralement d'un seul côté comme l'illustre la figure 3. Dans cet évidement 20 est logé un boisseau rotatif 22, réalisé par exemple en matière plastique transparente, apte à tourner à l'intérieur de l'évidement 20 autour de l'axe horizontal de ce dernier.

Pour permettre de commander la rotation du boisseau 22 à l'intérieur de l'évidement 20, une poignée 24 télémanipulable est fixée sur la face du boisseau 22 tournée vers l'extrémité ouverte de l'évidement 20, par exemple par des vis 26. Ces vis servent également à fixer sur la face correspondante du boisseau 22 une calotte d'étanchéité annulaire 28 qui obture de façon étanche l'évidement 20 par rapport à l'atmosphère contenue dans l'enceinte étanche 10.

Le boisseau rotatif 22 délimite intérieurement un réceptacle 30, de forme cylindrique, dont l'axe est orienté radialement par rapport à l'axe du boisseau 22. Ce réceptacle 30 débouche par l'une de ses extrémités directement sur la surface périphérique du boisseau 22, alors qu'il est prolongé à son extrémité opposée par un passage cylindrique coaxial 32 qui débouche lui aussi sur la surface périphérique extérieure du boisseau 22. Le diamètre du réceptacle 30 est légèrement supérieur au diamètre extérieur des récipients neufs R2, alors que le diamètre du passage 32 est plus petit.

Comme l'illustre en particulier la figure 2, le mouvement de rotation du boisseau 22 autour de son axe est normalement limité par une tige de blocage 34 qui est reçue de façon coulissante dans un alésage formé dans le corps 18, selon une direction orientée radialement par rapport à l'axe de pivotement du boisseau 22, cette direction étant horizontale dans le mode de réalisation représenté. La tige de blocage 34 est sollicitée radialement vers l'axe du boisseau 22 par un ressort de compression 36 qui prend appui sur une vis de guidage 38 fixée sur le corps 18. De cette manière, l'extrémité de la tige de blocage 34 fait normallement saillie dans une gorge en arc de cercle façonnée sur la surface périphérique extérieure du boisseau 22, cette gorge étant centrée sur l'axe de ce dernier.

La gorge précitée comporte une partie principale 40a relativement profonde, dans laquelle se trouve normalement l'extrémité de la tige de blocage 34. Cette partie principale 40a est prolongée par une partie complémentaire 40b, de moindre profondeur, dans laquelle peut être amenée l'extrémité correspondante de la tige de blocage 34, lorsque celle-ci est tirée radialement vers l'extérieur au moyen d'une poignée 42 télémanipulable, solidaire de l'extrémité de la tige 34 située à l'extérieur du corps 18.

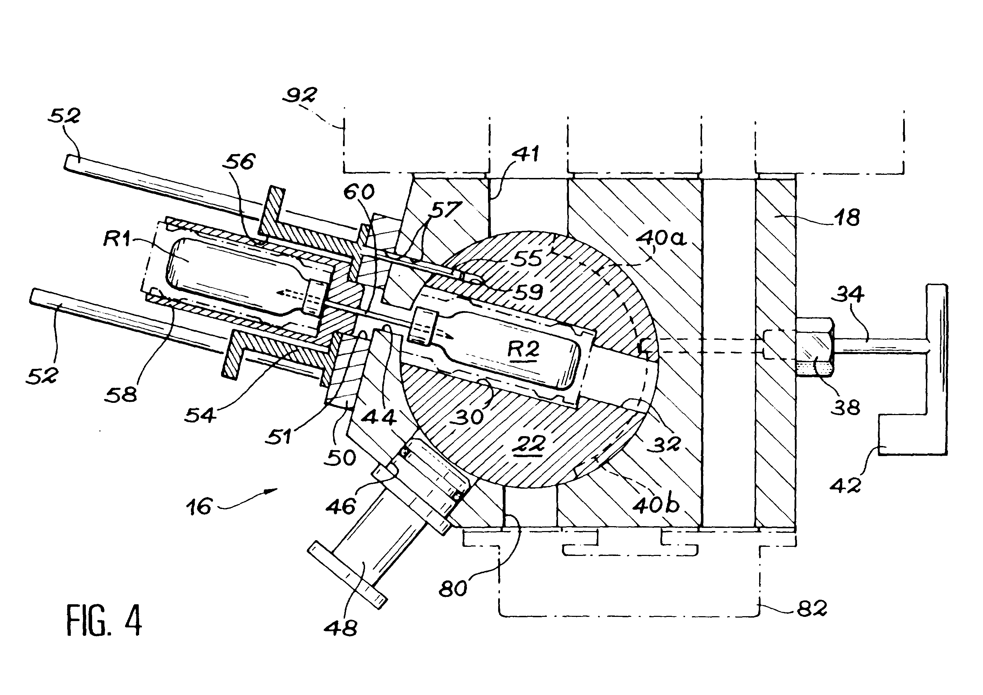

Lorsque l'extrémité de la tige de blocage 34 est située dans la partie principale 40a relativement profonde de la gorge, le boisseau rotatif 22 peut être déplacé entre une première position, illustrée sur les figures 2 et 3 et une deuxième position, illustrée sur la figure 4. Dans la première position, le réceptacle 30 est orienté verticalement de telle sorte que son extrémité ouverte débouche vers le haut en face d'un passage 41, de même diamètre que le réceptacle. Dans la deuxième position du boisseau 22, le réceptacle est orienté de telle sorte que son extrémité ouverte débouche en face d'un trou traversant 44, de faible diamètre, façonné dans le corps 18 selon une direction orientée radialement par rapport à l'évidement 20. Cette deuxième position peut être décalée d'un angle légèrement inférieur à 90° par rapport à la première position.

Lorsqu'un opérateur tire vers lui la poignée 42 de la tige de blocage 34, tout en continuant à agir sur la poignée 24 afin de faire tourner le boisseau 22 au-delà de la deuxième position précitée, il fait pénétrer l'extrémité de la tige 34 dans la partie complémentaire 40b de la gorge. L'opérateur peut ainsi amener le réceptacle 30 dans une troisième position, dans laquelle l'extrémité ouverte du réceptacle se trouve située en face d'un orifice d'évacuation exceptionnelle 46, de même diamètre que le réceptacle 30, et normalement obturé par un bouchon 48 télémanipulable. Cet orifice d'évacuation exceptionnelle 46 est situé dans la partie basse du corps 18, de façon à permettre une évacuation par gravité d'un récipient R2 placé dans le réceptacle 30, en cas de non fonctionnement du système de transfert pneumatique.

Une bille d'indexage 106 (figure 3), portée par une vis 108, est montée sur le corps 18, dans le fond de l'évidement 20, de façon à pouvoir pénétrer dans trois empreintes sphériques 110 formées sur la face correspondante du boisseau 22, lorsque celui-ci occupe l'une des trois positions précitées.

Comme l'illustre en particulier la figure 2, l'appareil 16 de transfert entre récipients comprend de plus un organe de guidage fixé sur une face plane du corps 18, dans laquelle est façonné le trou 44. Cet organe de guidage comprend une plaque 50, percée en son centre d'un trou 51 aligné avec le trou 44 dont le diamètre est légèrement plus grand. La plaque 50 est fixée au corps 18, par exemple au moyen de vis (non représentées). La plaque 50 supporte des colonnes de guidage 52, orientées parallèlement à l'axe du trou 44.

Sur ces colonnes de guidage 52 peut être reçu un coulisseau 54 apte à se déplacer le long des colonnes.

Le coulisseau 54 comporte, sur sa face tournée vers le corps 18, une tige 55 qui pénètre dans des trous alignés 57 formés dans la plaque 50 et dans le corps 18, ainsi que dans un trou 59 formé dans le boisseau 22, lorsque celui-ci est dans sa deuxième position (figure 4).

Le coulisseau 54 délimite intérieurement un logement cylindrique 56, normalement aligné avec le trou 44 et dans lequel peut être introduit un porte aiguille 58. Ce porte aiguille comporte une partie tubulaire, centrée sur l'axe du trou 44 lorsqu'il est reçu dans le logement 56, et un fond, prévu pour être tourné vers le trou 44 et que traverse de façon étanche, selon l'axe de ce trou, une aiguille 60. Cette aiguille 60 est effilée à chacune de ses extrémités, de façon à pouvoir perforer des opercules obturant respectivement le récipient pollué R1 qui contient la solution fluide à transférer et le récipient propre R2 dans lequel cette solution doit être transférée.

La partie tubulaire du porte aiguille 58 est dimensionnée de telle sorte que le récipient pollué R1 contenant la solution fluide à transférer peut y être introduit avec son opercule tourné vers l'aiguille 60. Cette introduction est généralement effectuée avant que le porte aiguille 58 ne soit introduit dans le logement 56 du coulisseau 54. Cette opération est effectuée à distance à l'aide de moyens de manutention appropriés équipant par ailleurs l'enceinte étanche 10. Ces moyens de manutention peuvent notamment être des télémanipulateurs.

Lorsque le récipient pollué R1 contenant la solution fluide à transférer a été placé dans le porte aiguille 58 de telle sorte que son opercule soit perforé par l'aiguille 60, le porte aiguille 58 est placé dans le logement 56 du coulisseau 54, qui est alors maintenu éloigné de la plaque 50. L'opérateur commande ensuite, toujours à distance, la rotation du boisseau 22, de façon à amener l'extrémité ouverte du réceptacle 30 en face du trou 44. Le récipient propre R2 introduit auparavant dans le réceptacle 30 se trouve alors disposé de telle sorte que son opercule soit placé en face du trou 44. L'opérateur déplace alors le coulisseau 54 dans lequel est placé le porte aiguille 58 portant le récipient pollué R1, vers le trou 44, jusqu'à ce que le coulisseau 54 arrive en butée contre la plaque 50. A ce moment, l'extrémité de l'aiguille 60 opposée à celle qui est plantée dans le récipient pollué R1 a perforé l'opercule du récipient propre R2 placé dans le réceptacle 30, comme l'illustre la figure 4.

Etant donné que l'intérieur du récipient R2 a été placé préalablement sous vide, un transfert de la solution fluide contenue initialement dans le récipient pollué R1 vers le récipient propre R2 s'effectue alors de façon automatique. Lorsque ce transfert est terminé, des opérations inverses des précédentes sont réalisées, toujours à distance, par l'opérateur, afin d'évacuer le récipient pollué R1 vers une poubelle située à l'intérieur de l'enceinte étanche 10 et de transférer le récipient propre R2 contenant alors la solution fluide à l'extérieur de cette enceinte.

L'introduction d'un récipient propre R2 dans le réceptacle 30 ainsi que son transfert hors de l'enceinte 10 après que la solution fluide y ait été introduite sont effectués par un tube 62 (figure 2) dont une partie située à l'intérieur de l'enceinte 10 est orientée verticalement, de façon à être raccordée par son extrémité inférieure sur le corps 18, dans le prolongement du passage 41 qui surplombe le réceptacle 30 lorsque ce dernier occupe sa première position. Les diamètres intérieurs du tube 62, du passage 41 et du réceptacle 30 sont identiques et légèrement supérieurs au diamètre extérieur des récipients propres R2, de façon à permettre le déplacement de ces derniers par injection d'air comprimé.

Au-dessus de l'appareil de transfert entre flacons 16, le tube 62 traverse une paroi 64 de l'enceinte étanche 10, avantageusement constituée par une partie démontable comme l'illustre schématiquement la figure 2. En se référant à nouveau à la figure 1, on voit que le tube 62 traverse ensuite la cloison supérieure de la cellule de confinement 14, pour être raccordé au-dessus de cette dernière sur un dispositif d'aiguillage 66. Ce dispositif d'aiguillage 66 permet de faire communiquer le tube 62 soit avec un tube 68 d'amenée de récipients neufs R2 sous vide, soit avec un tube 70 de transfert d'un récipient neuf R2 contenant une solution fluide.

Le tube 68 retraverse la cloison 14 de la cellule pour déboucher à son extrémité opposée dans un boisseau 72 d'introduction de récipients neufs sous vide, à l'opposé d'une tuyauterie 74 d'arrivée d'air. La tuyauterie 74 est alimentée en air au travers d'un dispositif 76, situé à l'extérieur de la cellule 14, permettant d'interrompre l'arrivée d'air et de mettre la tuyauterie 74 à la pression atmosphérique.

Le transfert des récipients neufs sous vide depuis le boisseau d'introduction 72 dans le réceptacle 30 est effectué en injectant de l'air par la canalisation 74, après avoir placé le dispositif d'aiguillage 66 et le boisseau 22 dans les positions appropriées.

Pour sa part, le tube de transfert 70 communique par son extrémité opposée au dispositif d'aiguillage 66 avec une boîte à gants (non représentée) dans laquelle différentes mesures physiques ou des analyses de radiométrie peuvent notamment être effectuées sur les échantillons fluides contenus dans les récipients R2.

Pour transférer chacun des récipients neufs R2 en dehors de l'enceinte étanche 10 après qu'un échantillon fluide ait été transféré dans ce récipient, on utilise une injection d'air dans le réceptacle 30, qui s'effectue par le passage 32, à l'opposé de l'extrémité ouverte du réceptacle. Ce passage 32 fait communiquer le fond du réceptacle 30 avec un passage 80 formé dans le corps 18, lorsque le boisseau 22 occupe sa première position. Ce passage 80 est axialement aligné avec le passage 41 formé dans le corps 18, dans le prolongement du tube 62. Une tubulure 82, fixée de façon étanche sur le corps 18, fait communiquer le passage 80 avec un deuxième passage vertical 84 formé dans le corps 18 et traversant ce dernier sur toute sa hauteur. Sur l'extrémité supérieure du passage 84 est raccordée de façon étanche une tuyauterie 86, qui s'étend verticalement à l'intérieur de l'enceinte étanche 10, parallèlement et à côté de la partie du tube 62 située dans cette enceinte.

La tuyauterie 86 traverse de façon étanche la paroi 64 de l'enceinte, ainsi que la paroi de la cellule 14 (figure 1), pour être raccordée à son extrémité opposée par une source d'air, par l'intermédiaire d'un dispositif 88 permettant d'interrompre l'arrivée d'air et de mettre à l'atmosphère la tuyauterie 86.

Lorsque de l'air est introduit par la tuyauterie 86, le récipient R2 présent dans le réceptacle 30 est transféré automatiquement par le tube 62 puis par le tube 70 vers la boîte à gants dans laquelle débouche ce dernier, lorsque le dispositif d'aiguillage 66 est placé dans la position appropriée.

Dans le mode de réalisation illustré sur les figures, le dispositif 16 de transfert entre cruchons est fixé de façon étanche sur une plaque horizontale 90 (figure 2) sur laquelle sont soudées les extrémités inférieures du tube 62 et de la tuyauterie 86.

Pour cela, une glissière 92 est fixée de façon étanche, par exemple par des vis 93, sur la face supérieure horizontale du corps 18. Cette glissière 92 est prévue pour venir s'accrocher, par un mouvement de coulissement horizontal selon une direction perpendiculaire au plan de la figure 2, sur la plaque 90. Des cames rotatives 94, d'axes horizontaux, sont montées dans la glissière 92 de façon à pouvoir être manoeuvrées à distance par des leviers 96. Chacune des cames 94 porte à son extrémité un galet 98 qui est en appui sur une barrette horizontale 99, reliée par deux tiges 100 à un patin d'appui horizontal 101. Les tiges 100 sont aptes à coulisser selon une direction verticale dans la glissière 92. Chacun des patins d'appui 101 peut ainsi venir en appui contre la face supérieure de la plaque 90 pour bloquer celle-ci contre la glissière, dans la position appropriée des cames 94. Un ressort 102 associé à chacune des tiges 100 tend à déplacer celles-ci vers le haut pour éloigner normalement les patins 101 de la plaque 90. Chacune des cames 94 peut être bloquée par une vis 104.

Bien entendu, l'invention n'est pas limitée au mode de réalisation qui vient d'être décrit à titre d'exemple, mais en couvre toutes les variantes. Ainsi, on comprendra notamment que le boisseau rotatif portant le réceptacle peut être remplacé par un tiroir coulissant.

## Revendications

1. Procédé pour transférer en dehors d'une enceinte étanche (10) un fluide contenu dans un premier récipient (R1), sans sortir ce récipient de l'enceinte et sans rompre l'étanchéité de cette dernière, consistant à :
- introduire un deuxième récipient (R2) sous vide, dans un réceptacle mobile (30) placé à l'intérieur de l'enceinte (10), par un tube (62) traversant de façon étanche une paroi de l'enceinte et débouchant dans le réceptacle lorsque ce dernier occupe une première position ;
- placer le réceptacle mobile (30) dans une deuxième position, dans laquelle une zone perforable du deuxième récipient est exposée à l'intérieur de l'enceinte, et perforer ladite zone, ainsi qu'une zone perforable du premier récipient (R1) au moyen des deux extrémités d'une même aiguille (60), de façon à réaliser un transfert automatique de fluide dans le deuxième récipient sous vide ; puis
- ramener le réceptacle mobile (30) dans sa première position et évacuer le deuxième récipient (R2) hors de l'enceinte (10) par ledit tube (62).

2. Procédé selon la revendication 1, dans lequel on introduit le deuxième récipient (R2) dans le réceptacle mobile et on évacue le deuxième récipient hors de l'enceinte par des moyens de transfert pneumatiques.

3. Installation pour transférer en dehors d'une enceinte étanche (10) un fluide contenu dans un premier récipient (12), sans sortir ce récipient de l'enceinte et sans rompre l'étanchéité de cette dernière, comprenant :
- un réceptacle (30) mobile à l'intérieur d'un corps (18) placé dans l'enceinte, entre une première position et une deuxième position ;
- un tube (62) traversant de façon étanche une paroi (64) de l'enceinte et raccordé sur le corps, de façon à déboucher dans le réceptacle lorsque ce dernier occupe sa première position, pour y introduire un deuxième récipient sous vide ;
- un trou (44) formé dans ledit corps (18) en un emplacement situé en face du réceptacle (30), lorsque ce dernier occupe sa deuxième position ;
- un porte aiguille (58) portant une aiguille (60) dont une première extrémité est apte à être plantée dans une zone perforable du premier récipient et dont une deuxième extrémité est apte à être plantée dans une zone perforable du deuxième récipient, au travers dudit trou, lorsque le réceptacle occupe sa deuxième position.

4. Installation selon la revendication 3, dans laquelle le corps (18) supporte, en face dudit trou, un organe de guidage (50,52) sur lequel un coulisseau (54), apte à recevoir le porte aiguille (58), peut se déplacer selon une direction confondue avec l'axe dudit trou.

5. Installation selon l'une quelconque des revendications 3 et 4, dans laquelle une tuyauterie (86) traversant de façon étanche ladite paroi (64) de l'enceinte comporte une première extrémité raccordée sur le corps (18), de façon à déboucher dans le réceptacle (30), à l'opposé dudit tube, lorsque le réceptacle occupe sa première position, et une deuxième extrémité raccordée sur des moyens d'introduction d'air, à l'extérieur de l'enceinte.

6. Installation selon la revendication 5, dans laquelle le tube (62) et la tuyauterie (86) comportent deux premières parties parallèles traversant ladite paroi (64) de l'enceinte et deux parties (41;80,82,84) formées au moins partiellement dans ledit corps (18), ce dernier étant fixé aux premières parties parallèles par des moyens de raccordement (90,92) étanches et démontables.

7. Installation selon l'une quelconque des revendications 3 à 6, dans laquelle le corps (18) comporte un orifice d'évacuation exceptionnelle (46) du deuxième récipient, normalement obturé par un bouchon (48), en face duquel peut être amené le réceptacle (30), dans une troisième position de ce dernier.

8. Installation selon la revendication 7, dans laquelle un organe de blocage (34) monté dans le corps (18) empêche normalement le passage du réceptacle (30) dans la troisième position, tant que ledit organe de blocage n'est pas actionné.

9. Installation selon l'une quelconque des revendication 3 à 8, dans laquelle le réceptacle mobile (30) est monté dans un boisseau rotatif (22), d'axe perpendiculaire à un axe dudit réceptacle.

10. Installation selon l'une quelconque des revendications 3 à 9, dans laquelle des moyens d'indexage (106) sont montés dans ledit corps, pour repérer chacune des positions.

## Claims

1. Process for transferring out of a tight enclosure (10) a fluid contained in a first container (R1), without removing the latter from the enclosure and without breaking the seal of the latter, comprising introducing a second container (R2) under vacuum into a mobile receptacle (30) placed within the enclosure (10), by a tube (62) tightly traversing a wall of the enclosure and issuing into the receptacle when the latter occupies a first position, placing the mobile receptacle (30) in a second position, in which a perforatable zone of the second container is exposed to the interior of the enclosure and perforating said zone, as well as a perforatable zone of the first container (R1) by means of the two ends of the same needle (60), so as to carry out an automatic transfer of the fluid into the second container under vacuum and then bringing the mobile container (30) into its first position and evacuating the second container (R2) out of the enclosure (10) by the said tube (62).

2. Process according to claim 1, wherein the second container (R2) is introduced into the mobile receptacle and the second container is discharged out of the enclosure by pneumatic transfer means.

3. Installation for the transfer out of a tight enclosure (10) of a fluid contained in a first container (12), without removing the latter from the enclosure and without breaking the seal of the latter, comprising a receptacle (30) mobile within a body (18) placed in the enclosure between a first position and a second position, a tube (62) tightly traversing a wall (64) of the enclosure and connected to the body, so as to issue into the receptacle when the latter occupies its first position for introducing into it a second container under vacuum, a hole (44) formed in the body (18) at a location facing the receptacle (30), when the latter occupies its second position and a needle holder (58) carrying a needle, whereof a first end can be fixed in a perforatable zone of the first container and whereof a second end can be fixed in a perforatable zone of the second container, through the said hole, when the receptacle occupies its second position.

4. Installation according to claim 3, wherein the body (18) supports, in front of the said hole, a guide member (50, 52) on which a slide (54), able to receive the needle holder (58), can travel in a direction coinciding with the axis of said hole.

5. Installation according to claim 3 or 4, wherein the pipe (86) tightly traversing the wall (64) of the enclosure has a first end connected to the body (18) so as to issue into the receptacle (30) opposite to the said tube, when the receptacle occupies its first position, and a second end connected to air introduction means outside the enclosure.

6. Installation according to claim 5, wherein the tube (62) and the pipe (86) have two first parallel parts traversing the said enclosure wall (64) and two parts (41; 80, 82, 84) at least partly formed in the said body (18), the latter being fixed to the first parallel parts by tight, dismantlable connecting means (90, 92).

7. Installation according to any one of the claims 3 to 6, wherein the body (18) has an exceptional discharge orifice (46) for the second container, which is normally sealed by a plug (48) and in front of which can be brought the receptacle (30) in a third position of the latter.

8. Installation according to claim 7, wherein a locking member (34) mounted in the body (18) normally prevents the passage of the receptacle (30) into the third position, when the locking member is not actuated.

9. Installation according to any one of the claims 3 to 8, wherein the mobile receptacle (30) is mounted in a rotary casing (22), whose axis is perpendicular to the receptacle axis.

10. Installation according to any one of the claims 3 to 9, wherein indexing means (106) are mounted in the said body in order to index each of the positions.

## Patentansprüche

1. Verfahren zum Transfer eines in einem ersten Rezipienten (R1) enthaltenen Fluidums aus einem Behälter (10), ohne diesen Rezipienten aus dem Behälter herauszunehmen und ohne die Dichtheit dieses letzteren zu unterbrechen, folgende Schritte umfassend:
- Einführen eines zweiten Rezipienten (R2) unter Vakuum in eine im Innern des Behälters (10) befindliche bewegliche Aufnahme (30) durch ein Rohr (62), das auf dichte Weise eine Wand des Behälters durchquert und in der Aufnahme mündet, wenn diese letztere eine erste Stellung einnimmt;
- die bewegliche Aufnahme (30) in eine zweite Stellung bringen, in der eine perforierbare Zone des zweiten Rezipienten dem Behälterinnern ausgesetzt ist und diese Zone sowie eine perforierbare Zone des ersten Rezipienten (Rl) mittels der beiden Enden von ein und derselben Nadel (60) perforieren, um einen automatischen Transfer des Fluidums in den zweiten Rezipienten unter Vakuum durchzuführen; dann
- die bewegliche Aufnahme (30) in ihre erste Stellung zurückbringen und den zweiten Rezipienten (R2) durch das genannte Rohr (62) aus den Behälter (10) entfernen.

2. Verfahren nach Anspruch 1, bei dem man den zweiten Rezipienten (R2) in die bewegliche Aufnahme einführt und man den zweiten Rezipienten durch pneumatische Transfereinrichtungen aus dem Behälter entfernt.

3. Vorrichtung zum Transfer eines in einem ersten Rezipienten (R1) enthaltenen Fluidums aus einem Behälter (10), ohne diesen Rezipienten aus dem Behälter herauszunehmen und ohne die Dichtheit dieses letzteren zu unterbrechen, umfassend:
- eine Aufnahme (30) im Innern eines Gehäuses (18), in dem Behälter befindlich, beweglich zwischen einer ersten Stellung und einer zweiten Stellung;
- ein Rohr (62), auf dichte Weise eine Wand (64) des Behälters durchquerend und angeschlossen an den Körper, so daß es in der Aufnahme mündet, wenn diese letztere ihre erste Stellung einnimmt, um dort einen zweiten Rezipienten unter Vakuum einzuführen;
- ein Loch (44), ausgebildet in dem genannten Gehäuse (18) an einer Stelle, die der Aufnahme (30) gegenübersteht, wenn diese ihre zweite Stellung einnimmt;
- einen Nadelhalter (58) mit einer Nadel (60), deren eines Ende eine perforierbare Zone des ersten Rezipienten durchstechen kann und deren zweites Ende eine perforierbare Zone des zweiten Rezipienten durchstechen kann, durch besagtes Loch hindurch, wenn die Aufnahme sich in ihrer zweiten Stellung befindet.

4. Vorrichtung nach Anspruch 3, bei der das Gehäuse (18) gegenüber diesem Loch eine Führungseinrichtung (50,52) trägt, in der ein Gleitstück (54), das den Nadelhalter (58) trägt, sich in einer Richtung verschieben läßt, die zusammenfällt mit der Achse des besagten Lochs.

5. Vorrichtung nach einem der Ansprüche 3 und 4, bei der eine Rohrleitung (86), die auf dichte Weise die Wand (64) des Behälters durchquert, mit einem ersten Ende mit dem Gehäuse (18) verbunden ist, so daß es in der Aufnahme (30) mündet, auf der dem genannten Rohr entgegengesetzten Seite, wenn die Aufnahme ihre erste Stellung einnimmt, und mit einem zweiten Ende außerhalb des Behälters mit Lufteinleitungseinrichtungen verbunden ist.

6. Vorrichtung nach Anspruch 5, bei der das Rohr (62) und die Rohrleitung (86) zwei parallele, die Wand (64) durchquerende erste Teilstücke umfassen, und zwei Teilstücke (41;80,82,84), die wenigstens teilweise in dem Gehäuse (18) ausgebildet sind, wobei dieses letztere durch dichte und demontierbare Verbindungseinrichtungen (90,92) an den parallelen ersten Teilstücken befestigt ist.

7. Vorrichtung nach einem der Ansprüche 3 bis 6, bei der das Gehäuse (18) eine normalerweise durch einen Stopfen (48) verschlossene Sonderöffnung (46) zum Ausstoßen des zweiten Rezipienten umfaßt, wobei die Aufnahme (30) in eine dritte Stellung gebracht werden kann, gegenüber dieser Sonderöffnung (46).

8. Vorrichtung nach Anspruch 7, bei der ein im Gehäuse (18) vorgesehenes Blockierorgan (34) normalerweise den Übergang der Aufnahme (30) in die dritte Stellung verhindert, solange dieses Blockierorgan nicht betätigt wird.

9. Vorrichtung nach einem der Ansprüche 3 bis 8, bei dem die bewegliche Aufnahme (30) in einem Hahnkegel (22) mit einer zur Achse dieser Aufnahme senkrechten Achse vorgesehen ist.

10. Vorrichtung nach einem der Ansprüche 3 bis 9, bei der in dem genannten Gehäuse Indexierungseinrichtungen (106) angebracht sind, um jede dieser Stellungen festzulegen.
